# EUROPÄISCHE PATENTSCHRIFT

(11) **EP 0 931 067 B1**
(45) Veröffentlichungstag und Bekanntmachung des Hinweises auf die Patenterteilung: **26.06.2002**
(21) Anmeldenummer: 97944823.0
(22) Anmeldetag: 03.09.1997
(51) Int. Cl.: C07D 213/38, A61K 31/495, C07D 401/12, A61K 31/505, C07D 405/12, C07D 409/12, C07D 295/06, C07D 239/42, C07D 409/14

(54) **PIPERAZIN-DERIVATE MIT EINER SELEKTIVITÄT FÜR DEN D-4 REZEPTOR**
D4 RECEPTOR SELECTIVITY PIPERAZINE DERIVATIVES
DERIVES DE PIPERAZINE PRESENTANT UNE SELECTIVITE POUR LE RECEPTEUR D4

(30) Priorität: 13.09.1996 DE 19637237
(43) Veröffentlichungstag der Anmeldung: 28.07.1999
(73) Patentinhaber: MERCK PATENT GmbH, 64293 Darmstadt (DE)
(72) Erfinder: ARLT, Michael, D-64342 Seeheim-Jugenheim (DE); BÖTTCHER, Henning, D-64287 Darmstadt (DE); BARTOSZYK, Gerd, D-64331 Weiterstadt (DE); SEYFRIED, Christoph, D-64342 Seeheim-Jugenheim (DE)
(86) Internationale Anmeldenummer: EP9704789
(87) Internationale Veröffentlichungsnummer: WO9811068

(56) Entgegenhaltungen:
- EP-A- 0 745 598
- WO-A-94/20497
- WO-A-94/21630
- WO-A-94/22839
- WO-A-95/17400
- WO-A-96/16040

## Beschreibung

Die Erfindung betrifft Piperazin-Derivate der Formel I 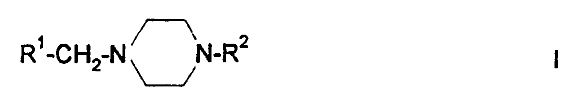 worin
- R¹: einfach durch Ph oder 2- oder 3-Thienyl substituiertes Pyridyl oder Phenyl,
- R2: Ph' oder Het,
- Ph und Ph': jeweils unabhängig voneinander Phenyl, wobei beide Reste jeweils unsubstituiert oder ein-, zwei- oder dreifach durch F, Cl, Br, I, OH, OA, A, CF₃, NO₂, CN, COA, CONH₂, CONHA, CONA₂ oder 2- oder 3-Thienyl substituiert sein können,
- Het: einen gesättigten, teilweise oder vollständig ungesättigten mono- oder bicyclischen heterocyclischen Rest mit 5 bis 10 Ringgliedern, wobei 1 oder 2 N- und/oder 1 oder 2 O-Atome vorliegen können und der heterocyclische Rest ein- oder zweifach durch F, Cl, Br, I, OA, CF₃, A oder NO₂ substituiert sein kann
und
- A: Alkyl mit 1 bis 6 C-Atomen
bedeuten
sowie deren physiologisch unbedenkliche Salze.

Der Erfindung lag die Aufgabe zugrunde, neue Verbindungen aufzufinden, die zur Herstellung von Arzneimitteln verwendet werden können.

Andere Piperazinderivate mit einer Selektivität für den D4-Rezeptor sind in den nachstehenden Dokumenten beschrieben:
EP-A-0 745 598, WO-A-94 20497, WO-A-96 16040, WO-A-96 21630 und WO-A-94 22839.

Es wurde gefunden, daß die Verbindungen der Formel I und ihre physiologisch unbedenklichen Säureadditionssalze wertvolle pharmakologische Eigenschaften besitzen. Die Verbindungen der Formel I sind Dopaminliganden mit einer Selektivität für den D4-Rezeptor, im Vergleich zu D2 und D3 Rezeptoren (Methode analog zu Creese et al, European J. Pharmacol. 46, 377-381 (1977); mit ³H-Spiroperidol als Liganden für Dopaminrezeptoren und klonierten, humanen Dopamin D4-, D3- und D2-Rezeptoren(Bezugsquelle: Receptor Biology Inc., Baltimore MD 21227, USA)). Die Verbindungen sind geeignet für die Behandlung von Schizophrenie, kognitiven Defiziten, Angst, Depressionen, Übelkeit, tardive Dyskinesie, Magen-Darm-Trakt-Störungen oder Parkinsonismus. Sie zeigen Wirkungen auf das Zentralnervensystem, vor allem zusätzliche 5-HT_{1A}-agonistische und 5-HT-Reuptake hemmende Wirkungen. Die Verbindungen zeigen ferner serotonin-agonistische und -antagonistische Eigenschaften. Sie hemmen die Bindung von tritiierten Serotoninliganden an hippocampale Rezeptoren (Cossery et al., European J. Pharmacol. 140 (1987), 143-155). Außerdem treten Veränderungen der DOPA-Akkumulation im Striatum und der 5-HTP-Akkumulation in N. Raphe auf (Seyfried et al., European J. Pharmacol. 160 (1989), 31-41). Weiterhin treten analgetische und blutdrucksenkende Wirkungen auf; so wird bei kathetertragenden wachen, spontan hypertonen Ratten (Stamm SHR/Okamoto/NIH-MO-CHB-Kisslegg; Methode vgl. Weeks und Jones, Proc. Soc. Exptl. Biol. Med. 104 (1960), 646-648) der direkt gemessene Blutdruck nach peroraler Gabe der Verbindungen gesenkt. Ebenso eignen sie sich zur Prophylaxe und zur Bekämpfung der Folgen cerebraler Infarktgeschehen (apoplexia cerebri) wie Schlaganfall und cerebraler Ischämien.

Verbindungen der Formel I und ihre physiologisch unbedenklichen Säureadditionssalze können daher als Arzneimittelwirkstoffe für Anxiolytika, Antidepressiva, Antipsychotika, Neuroleptika und/oder Antihypertonika und auch als Zwischenprodukte zur Herstellung anderer Arzneimittelwirkstoffe verwendet werden.

Gegenstand der Erfindung sind Piperazinderivate der Formel I sowie ihre physiologisch unbedenklichen Säureadditionssalze.

Der Rest A bedeutet Alkyl mit 1, 2, 3, 4, 5 oder 6, insbesondere 1 oder 2 C-Atomen, vorzugsweise Methyl, ferner auch Ethyl, n-Propyl, Isopropyl, n-Butyl, sek.-Butyl oder tert.-Butyl. OA ist vorzugsweise Methoxy, ferner auch Ethoxy, n-Propoxy, Isopropoxy, n-Butoxy, Isobutoxy, sek.-Butoxy oder tert.-Butoxy. NHA ist vorzugsweise Methylamino, ferner Ethylamino, Isopropylamino, n-Butylamino, Isobutylamino, sek.-Butylamino oder tert.-Butylamino. NA₂ bedeutet vorzugsweise Dimethylamino, ferner N-Ethyl-N-methylamino, Diethylamino, Di-n-propylamino, Diisopropylamino oder Di-nbutylamino. Daraus resultierend bedeutet CO-NHA vorzugsweise N-Methylcarbamoyl oder N-Ethyl-carbamoyl; CO-NA₂ vorzugsweise N,N-Dimethylcarbamoyl oder N,N-Diethylcarbamoyl.

Der Rest R¹ bedeutet vorzugsweise unsubstituiertes oder einfach durch 3-Thienyl substituiertes Phenyl, unsubstituiertes oder einfach durch OA, CN, CF₃, F, Br oder Cl substituiertes Biphenyl oder 2-, 3- oder 4-Pyridyl, welches besonders bevorzugt durch 3-Thienyl, Phenyl, p- m- oder o-F-Phenyl substituiert sein kann. Sofern R¹ substituiertes oder unsubstituiertes Pyridyl bedeutet, so ist der 3-Pyridyl-Rest bevorzugt.

Der Rest R² bedeutet vorzugsweise unsubstituiertes oder ein-, zwei- oder dreifach durch F, Cl, Br, OH, OA, A, CONH₂, COA, CF₃, CN und/oder NO₂ substituiertes Phenyl oder entsprechend substituiertes Het, wobei Het vorzugsweise 1,4-Benzodioxan, 2-, 3- oder 4-Pyridyl, 2-, 4-, 5- oder 6-Pyrimidinyl, 3- oder 4-Pyridazinyl, 2- oder 3-Pyrazinyl sein kann.

Für die gesamte Erfindung gilt, daß sämtliche Reste wie z.B. Ph, die in einem Molekül mehrfach auftreten können, gleich oder verschieden sein können.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln la bis lk ausgedrückt werden, die der Formel I entsprechen und worin die nicht näher bezeichneten Reste die bei der Formel I angegebene Bedeutung haben, worin jedoch
- in Ia: R¹ in 5-Position substituiertes 3-Pyridyl ist;
- in Ib: R² 2-Pyrimidinyl ist;
- in Ic: R¹ Phenyl und R² unsubstituiertes oder einfach substituiertes Pyridyl oder Pyrimidinyl ist;
- In Id: R¹ Biphenyl und R² unsubstituiertes oder ein-, zwei- oder dreifach substituiertes Phenyl ist;
- in Ie: R¹ Biphenyl und R² unsubstituiertes oder einfach substituiertes 1,4-Benzodioxanyl, Benzofuranyl, Pyridyl, Pyrazinyl, Pyridazinyl oder Pyrimidinyl ist;
- in If: R¹ eine in la oder lb angegebene Bedeutung hat und R² ein-, zwei- oder dreifach substituiertes Phenyl bedeutet;
- in Ig: R¹ eine in la oder lb angegebene Bedeutung besitzt und R² einfach substituiertes oder unsubstituiertes Pyridyl, Pyrimidinyl, Pyrazinyl oder Pyridazinyl bedeutet;
- in Ih: R² 2-Pyrimidinyl ist und R¹ meta-substituiertes Phenyl oder in 5-Position substituiertes Pyrid-3-yl bedeutet;
- in Ii: R² 2-Pyrimidinyl ist und R¹ substituiertes Phenyl oder substituiertes Pyrid-3-yl bedeutet, wobei die Substituenten vorzugsweise OCH₃, F oder 2- oder 3-Thienyl sind;
- in Ik: R¹ eine in la angegebene Bedeutung hat und der Substituent Phenyl oder o- oder p-Fluorphenyl ist.

Gegenstand der Erfindung ist ferner ein Verfahren zur Herstellung von Piperazin-Derivaten der Formel I sowie von deren Salzen, dadurch gekennzeichnet, daß man eine Verbindung der Formel II 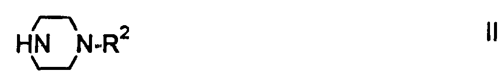 worin R² die angegebene Bedeutung besitzt, mit einer Verbindung der Formel III

R¹-CH₂-L III

worin
- L: Cl, Br, I, OH, O-CO-A, O-CO-Ph, O-SO₂-Ar, wobei Ar für Phenyl oder Tolyl und A für Alkyl stehen, oder eine andere reaktionsfähig veresterte OH-Gruppe oder leicht nucleophil substituierbare Abgangsgruppe bedeutet und
- R¹: die angegebene Bedeutung hat,
umsetzt,
oder daß man eine Verbindung der Formel IV

H₂N-R² IV

worin
- R²: die angegebene Bedeutung hat,
mit einer Verbindung der Formel V 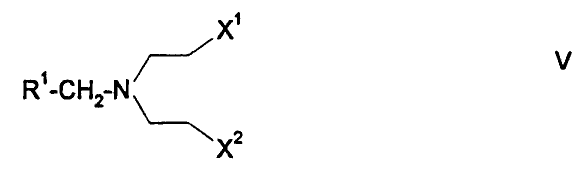 worin
- X¹ und X²: gleich oder verschieden sein können und Cl, Br, I, OH oder eine reaktionsfähige funktionell abgewandelte OH-Gruppe bedeuten, R¹ die angegebene Bedeutung hat,
umsetzt,
oder daß man eine Verbindung der Formel VI 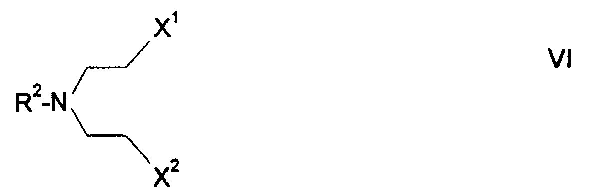 worin
- R², X¹ und X¹: die bereits angegebenen Bedeutungen haben,
mit einer Verbindung der Formel VII

R¹-CH₂-NH₂ VII

worin
- R¹: die angegebene Bedeutung hat,
umsetzt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-Cund/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt,
und/oder daß man gegebenenfalls einen Rest R¹ und/oder R² in einen anderen Rest R¹ und/oder R² umwandelt, indem man z.B. eine OA-Gruppe unter Bildung einer OH-Gruppe spaltet und/oder eine CN-, COOH-, COOA-Gruppe derivatisiert und/oder daß man z.B. ein primäres oder sekundäres N-Atom alkyliert und/oder eine erhaltene Base oder Säure der Formel I durch Behandeln mit einer Säure oder Base in eines ihrer Salze umwandelt.

Die Herstellung der Verbindungen der Formel I erfolgt im übrigen nach an sich bekannten Methoden, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York; DE-OS 41 01 686) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

Die Verbindungen der Formel II und III sind in der Regel bekannt; die nicht bekannten Verbindungen der Formeln II und III können leicht analog zu den bekannten Verbindungen hergestellt werden.

Die Piperazin-Derivate der Formel II sind größtenteils bekannt und können beispielsweise durch Umsetzung von Bis-(2-chlorethyl)-amin oder dem entsprechenden Ammoniumchlorid mit Anilin-, Aminonaphthalin- oder Aminobiphenyl-Derivaten der Formel Ph'-NH₂ bzw. mit Amino-substituierten heterocyclischen Verbindungen der Formel Het-NH₂ hergestellt werden.

Die Pyridine, Benzylverbindungen, Naphthalin- oder Biphenyl-Derivate der Formel III sind in der Regel bekannt und können meist käuflich erworben werden. Ferner kann man die Verbindungen durch elektrophile oder in bestimmten Fällen auch nukleophile aromatische Substitutionen aus bekannten Verbindungen herstellen. Insbesondere lassen sich Verbindungen der Formel III jedoch herstellen, indem man bei Verbindungen der Formel R¹-CH₃, z.B. durch radikalische Substitution, einen Rest L einführt, oder in einer Verbindung der Formel III einen bestehenden Rest L in einen anderen Rest L umwandelt. So kann man z.B. eine OH-Gruppe verestern oder nucleophil durch Halogen substituieren.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, N-Butanol; Ether wie Tetrahydrofuran (THF) oder Dioxan; Amide wie Dimethylformamid (DMF) oder N-Methyl-pyrrolidion; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses Piperazin-Derivates der Formel II kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Desweiteren kann man Verbindungen der Formel I herstellen, indem man Amine der Formel IV mit Verbindungen der Formel V umsetzt.

Die Amine der Formel IV sind in der Regel bekannt. Ferner können sie z.B. durch Reduktion entsprechender Nitroverbindungen hergestellt werden, wobei die Nitroverbindungen, wie allgemein bekannt, durch Nitrierung am Aromaten hergestellt werden können.

Verbindungen der Formel V sind z.B. herstellbar durch Reduktion von Diestern der Formel AlkylOOC-CH₂-N(CH₂R¹)-CH₂-COOalkyl zu Verbindungen der Formel HO-CH₂-CH₂-N(CH₂R¹)-CH₂-CH₂OH und gegebenenfalls anschließende Umsetzung mit SOCl₂ bzw. PBr₃.

Verbindungen der Formel V lassen sich auch durch Umsetzung von sekundären Aminen der Formel HN(CH₂-CH₂X¹)(CH₂-CH₂-X²) mit Halogeniden der Formel R¹-CH₂-Hal (Hal = Cl, Br) herstellen.

Ferner kann man zur Herstellung von Verbindungen der Formel I auch Verbindungen der Formel VI mit Aminen der Formel VII umsetzen. Die Verbindungen der Formel VI sind strukturell den Verbindungen der Formel V ähnlich und können analog hergestellt werden. Das gleiche gilt für die Verbindungen der Formel VII im Hinblick auf die Amine der Formel V. Die Amine der Formel VII können zudem noch durch an sich bekannte Verfahren zur Synthese primärer Amine, wie z.B. die Gabriel-Synthese, hergestellt werden.

Die Umsetzung der Verbindungen IV und V bzw. VI mit VII verläuft nach Methoden wie sie für die Alkylierung von Aminen aus der Literatur bekannt sind und bereits oben angegeben werden.

Es ist ferner möglich, eine Verbindung der Formel I zu erhalten, indem man ein Vorprodukt, das anstelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen bzw. zusätzlichen Bindungen enthalten, reduktiv in eine Verbindung der Formel I überzuführen; dabei können gleichzeitig Substituenten in der Gruppe I, die in der Ausgangsverbindung enthalten sind, reduziert werden. Vorzugsweise bedient man sich hierzu des nascierenden Wasserstoffs oder komplexer Metallhydride, ferner der Reduktion nach Wolff-Kishner sowie der Reduktionen mit Wasserstoffgas unter Übergangsmetallkatalyse.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall gelöst in einem Alkohol wie Ethanol, Isopropanol, Butanol, Amyl- oder Isoamylalkohol oder Phenol. Man kann ferner eine Aluminium-Nickel-Legierung in alkalisch-wäßriger Lösung, gegebenenfalls unter Zusatz von Ethanol, verwenden. Auch Natrium- oder Aluminiumamalgam in wäßrig-alkoholischer oder wäßriger Lösung sind zur Erzeugung des nascierenden Wasserstoffs geeignet. Die Umsetzung kann auch in heterogener Phase durchgeführt werden, wobei man zweckmäßig eine wäßrige und eine Benzol- oder Toluol-Phase verwendet.

Als Reduktionsmittel können ferner besonders vorteilhaft komplexe Metallhydride, wie LiAlH₄, NaBH₄, Diisobutylaluminiumhydrid oder NaAl(OCH₂CH₂OCH₃)₂H₂ sowie Diboran eingesetzt werden, falls erwünscht unter Zusatz von Katalysatoren wie BF₃, AlCl₃ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für die Reduktion mit NaBH₄ sind in erster Linie Alkohole wie Methanol oder Ethanol, ferner Wasser sowie wäßrige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich -CO-Gruppen in Säureamiden mit LiAlH₄ in THF bei Temperaturen zwischen etwa 0 und 66° zu CH₂-Gruppen reduzieren.

Es ist ferner möglich, eine oder mehrere Carbonylgruppen nach der Methode von Wolff-Kishner zu CH₂-Gruppen zu reduzieren, z.B. durch Behandlung mit wasserfreiem Hydrazin in absolutem Ethanol unter Druck bei Temperaturen zwischen etwa 150 und 250°. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel wie Diethylenglykol oder Triethylenglykol, in Gegenwart von Alkali, wie Natriumhydroxid, umsetzt. Das Reaktionsgemisch wird in der Regel etwa 3-4 Stunden gekocht.

Anschließend wird das Wasser abdestilliert und das gebildete Hydrazon bei Temperaturen bis zu etwa 200° zersetzt. Die Wolff-Kishner-Reduktion kann auch bei Raumtemperatur in Dimethylsulfoxid mit Hydrazon ausgeführt werden.

Darüber hinaus ist es möglich, bestimmte Reduktionen durch Verwendung von H₂-Gas unter katalytischer Wirkung von Übergangsmetallen, wie z.B. Raney-Ni oder Pd durchzuführen. Man kann auf diese Weise z.B. Cl, Br, I, SH oder in bestimmten Fällen auch OH-Gruppen durch Wasserstoff ersetzen. Ebenso können Nitrogruppen durch katalytische Hydrierung mit Pd/H₂ in Methanol in NH₂-Gruppen umgewandelt werden.

Verbindungen, die sonst der Formel I entsprechen, aber anstelle eines oder mehrerer H-Atome eine oder mehrere solvolysierbare Gruppe(n) enthalten, können zu den Verbindungen der Formel I solvolysiert, insbesondere hydrolysiert werden.

Die Ausgangsstoffe für die Solvolyse sind beispielsweise erhältlich durch Reaktion von Verbindungen der Formel II, die der Formel III entsprechen, aber anstelle eines oder mehrerer H-Atome eine oder mehrere solvolysierbare Gruppe(n) enthalten.

Weiterhin kann man eine Verbindung der Formel I nach an sich bekannten Methoden in eine andere Verbindung der Formel I umwandeln.

Verbindungen der Formel I, worin R¹ einen durch CONH₂, CONHA oder CONA₂ substituierten Rest bedeutet, können durch Derivatisierung entsprechender substituierter Verbindungen der Formel I durch partielle Hydrolyse erhalten werden. Ferner ist es möglich, Cyan-substituierte Verbindungen der Formel I zunächst zu Säuren zu hydrolysieren und die Säuren mit primären oder sekundären Aminen zu amidieren. Bevorzugte ist die Umsetzung der freien Carbonsäure mit dem Amin unter den Bedingungen einer Peptidsynthese. Diese Reaktion gelingt vorzugsweise in Gegenwart eines Dehydratisierungsmittels, z.B. eines Carbodiimids wie Dicyclohexylcarbodiimid oder N-(3-Dimethylaminopropyl)-N-ethyl-carbodiimid, ferner Propanphosphonsäureanhydrid (vgl. Angew. Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40°, vorzugsweise zwischen 0 und 30°.

Besonders günstig ist es aber auch in umgekehrter Weise, durch Wasserabspaltung, ausgehend von den Amiden, z.B. mittels Trichloracetylchlorid/Et₃N [Synthesis (2), 184 (1985)] oder mit POCl₃ (J. Org. Chem. 26, 1003 (1961)), die Nitrile herzustellen.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure; Benzolsulfonsäure, p-Toluolsulfonsäure, Naphthalin-monound -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen. In jenen Fällen, wo die Verbindungen der Formel I über freie Säuregruppen verfügen, kann durch Behandlung mit Basen ebenfalls eine Salzbildung erreicht werden. Als Basen eignen sich Alkalimetallhydroxide, Erdalkalimetallhydroxide oder organische Basen in Form von primären, sekundären oder tertiären Aminen.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositoren, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenden Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten verwendet werden. Sie eignen sich zur Behandlung von Erkrankungen des Zentralnervensystems wie Spannungszuständen, Depressionen, Angstzuständen, Schizophrenie, Magen-Darm-Trakt-Störungen, Übelkeit, tardiven Dyskinesie, Parkinsonismus und/oder Psychosen und von Nebenwirkungen bei der Behandlung der Hypertonie (z.B. mit α-Methyldopa). Ferner können die Verbindungen in der Endokrinologie und Gynäkologie Verwendung finden, z.B. zur Therapie von Akromegalie, Hypogonadismus, sekundärer Amenorrhoe, prämenstruellem Syndrom, unerwünschter puerperaler Laktation, weiterhin zur Prophylaxe und Therapie cerebraler Störungen (z.B. Migräne), insbesondere in der Geriatrie ähnlich wie gewisse Ergot-Alkaloide und zur Bekämpfung der Folgen cerebraler Infarktgeschehen (Apoplexia cerebri), wie Schlaganfall und cerebraler Ischämien.

Dabei werden die erfindungsgemäßen Substanzen in der Regel in Analogie zu bekannten, im Handel befindlichen Präparaten (z.B. Bromocriptin, Dihydroergocornin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen liegen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben. Rf-Werte wurden dünnschichtchromatographisch an Kieselgel erhalten. M⁺+1-Werte wurden durch FAB-MS (Fast-Atom-Bombardment-Massen-spektroskopie) ermittelt.

### Beispiel 1

Man löst 2,04 g 3-Chlormethyl-5-phenyl-pyridin ("A") [erhältlich z.B. durch radikalische Chlorierung von 3-Methyl-5-phenyl-pyridin] sowie 1,62 g 1-Phenyl-piperazin in 200 ml Acetonitril und rührt sechs Stunden bei Raumtemperatur. Nach üblicher Aufarbeitung erhält man das 1-Phenyl-4-[(5-phenyl-3-pyridyl)-methyl]-piperazin, F. 83-85°.

Analog erhält man durch Umsetzung von "A"
mit 1-(2-Fluor-phenyl)-piperazin das 1-(2-Fluor-phenyl)-4-[(5-phenyl-3-pyridyl)-methyl]-piperazin, Trihydrochlorid, F. 217-219°;
mit 1-(2-Methoxy-phenyl)-piperazin das 1-(2-Methoxy-phenyl)-4-[(5-phenyl-3-pyridyl)-methyl]-piperazin, Trihydrochlorid-Dihydrat, F. 235-236°;
mit 1-(2-Pyridyl)-piperazin das 1-(2-Pyridyl)-4-[(5-phenyl-3-pyridyl)-methyl]-piperazin, F. 103-105°;
mit 1-(3-Trifluormethyl-phenyl)-piperazin das 1-(3-Trifluormethyl-phenyl)-4-[(5-phenyl-3-pyridyl)-methyl]-piperazin, Trihydrochlorid, F. 216-219°.

Analog erhält man durch Umsetzung von 2-Chlormethyl-4-phenyl-pyridin
mit 1-Phenyl-piperazin das 1-Phenyl-4-[(4-phenyl-2-pyridyl)-methyl]-piperazin;
mit 1-(2-Fluor-phenyl)-piperazin das 1-(2-Fluor-phenyl)-4-[(4-phenyl-2-pyridyl)-methyl]-piperazin;
mit 1-(2-Methoxy-phenyl)-piperazin das 1-(2-Methoxy-phenyl)-4-[(4-phenyl-2-pyridyl)-methyl]-piperazin;
mit 1-(2-Pyridyl)-piperazin das 1-(2-Pyridyl)-4-[(4-phenyl-2-pyridyl)-methyl]-piperazin;
mit 1-(3-Trifluormethyl-phenyl)-piperazin das 1-(3-Trifluormethyl-phenyl)-4-[(4-phenyl-2-pyridyl)-methyl]-piperazin;
mit 1-(2-Pyrimidinyl)-piperazin das 1-(2-Pyrimidinyl)-4-[(4-phenyl-2-pyridyl)-methyl]-piperazin.

Analog erhält man durch Umsetzung von 2-Chlormethyl-4-(4-fluorphenyl)-pyridin
mit 1-Phenyl-piperazin das 1-Phenyl-4-[(4-(4-fluorphenyl)-2-pyridyl)-methyl]-piperazin;
mit 1-(2-Fluor-phenyl)-piperazin das 1-(2-Fluor-phenyl)-4-[(4-(4-fluorphenyl)-2-pyridyl)-methyl]-piperazin;
mit 1-(2-Methoxy-phenyl)-piperazin das 1-(2-Methoxy-phenyl)-4-[(4-(4-fluorphenyl)-2-pyridyl)-methyl]-piperazin;
mit 1-(2-Pyridyl)-piperazin das 1-(2-Pyridyl)-4-[(4-(4-fluorphenyl)-2-pyridyl)-methyl]-piperazin;
mit 1-(3-Trifluormethyl-phenyl)-piperazin das 1-(3-Trifluormethyl-phenyl)-4-[(4-(4-fluorphenyl)-2-pyridyl)-methyl]-piperazin;
mit 1-(2-Pyrimidinyl)-piperazin das 1-(2-Pyrimidinyl)-4-[(4-(4-fluorphenyl)-2-pyridyl)-methyl]-piperazin, Trihydrochlorid-Dihydrat, F. 193-195°;
mit 1-(5-Fluor-pyrimidin-2-yl)-piperazin das 1-(5-Fluor-pyrimidin-2-yl)-4-[(4-(4-fluorphenyl)-2-pyridyl)-methyl]-piperazin.

### Beispiel 2

Analog Beispiel 1 erhält man ausgehend von 1,10 g 3-Chlormethyl-5-(4-fluorphenyl)-pyridin ("B") [erhältlich z. B. durch radikalische Chlorierung von 3-Methyl-5-(4fluor-phenyl)-pyridin] durch Umsetzung mit 0,82 g 1-(2-Pyrimidinyl)-piperazin, in 200 ml Acetonitril bei Raumtemperatur, nach üblicher Aufarbeitung das 1-(2-Pyrimidinyl)-4-[(5-(4-fluorphenyl)-3-pyridyl)-methyl]-piperazin, F. 97-98°.

Analog erhält man durch Umsetzung von "B"
mit 1-(1,4-Benzodioxan-6-yl)-piperazin das 1-(1,4-Benzodioxan-6-yl)-4-[(5-(4-fluorphenyl)-3-pyridyl)-methyl]-piperazin, Trihydrochlorid, F. 256-259°;
mit 1-(4-Nitro-phenyl)-piperazin das 1-(4-Nitro-phenyl)-4-[(5-(4-fluorphenyl)-3-pyridyl)-methyl]-piperazin, Dihydrochlorid, F. 264°;
mit 1-(3,5-Dichlor-4-methoxy-phenyl)-piperazin das 1-(3,5-Dichlor-4-methoxy-phenyl)-4-[(5-(4-fluorphenyl)-3-pyridyl)-methyl]-piperazin, Dihydrochlorid, F. 163°;
mit 1-(4-Methoxy-phenyl)-piperazin das 1-(4-Methoxy-phenyl)-4-[(5-(4-fluorphenyl)-3-pyridyl)-methyl]-piperazin, Trihydrochlorid, F. 211°;
mit 1-(3,4-Dimethoxy-phenyl)-piperazin das 1-(3,4-Dimethoxy-phenyl)-4-[(5-(4-fluorphenyl)-3-pyridyl)-methyl]-piperazin, Trihydrochlorid F. 244°;
mit 1-(2-Fluor-phenyl)-piperazin das 1-(2-Fluor-phenyl)-4-[(5-(4-fluorphenyl)-3-pyridyl)-methyl]-piperazin, Dihydrochlorid F. 210°;
mit 1-(3,5-Dimethyl-4-methoxy-phenyl)-piperazin das 1-(3,5-Dimethyl-4-methoxy-phenyl)-4-[(5-(4-fluorphenyl)-3-pyridyl)-methyl]-piperazin, Trihydrochlorid, F. 251°;
mit 1-(2-Nitro-phenyl)-piperazin das 1-(2-Nitro-phenyl)-4-[(5-(4-fluorphenyl)-3-pyridyl)-methyl]-piperazin, Dihydrochlorid, F. 247°;
mit 1-(3-Chlor-5-trifluormethyl-pyrid-2-yl)-piperazin das 1-(3-Chlor-5-trifiuormethyl-pyrid-2-yl)-4-[(5-(4-fluorphenyl)-3-pyridyl)-methyl]-piperazin, Dihydrochlorid, F. 153°;
mit 1-(3-Methoxy-phenyl)-piperazin das 1-(3-Methoxy-phenyl)-4-[(5-(4-fluorphenyl)-3-pyridyl)-methyl]-piperazin, Trihydrochlorid, F. 232°;
mit 1-(2-Hydroxy-phenyl)-piperazin das 1-(2-Hydroxy-phenyl)-4-[(5-(4-fluorphenyl)-3-pyridyl)-methyl]-piperazin, Dihydrochlorid, F. 239°;
mit 1-(2-Pyrazinyl)-piperazin das 1-(2-Pyrazinyl)-4-[(5-(4-fluorphenyl)-3-pyridyl)-methyl]-piperazin, Dihydrochlorid-Hydrat, F. 140°;
mit 1-(4-Fluor-phenyl)-piperazin das 1-(4-Fluor-phenyl)-4-[(5-(4-fluorphenyl)-3-pyridyl)-methyl]-piperazin, Dihydrochlorid, F. 181°;
mit 1-(3-Trifluormethyl-4-chlor-phenyl)-piperazin das 1-(3-Trifluormethyl-4-chlor-phenyl)-4-[(5-(4-fluorphenyl)-3-pyridyl)-methyl]-piperazin, Sesquihydrochlorid, F. 230°;
mit 1-(2-Methyl-phenyl)-piperazin das 1-(2-Methyl-phenyl)-4-[(5-(4-fluorphenyl)-3-pyridyl)-methyl]-piperazin, Dihydrochlorid, F. 258°;
mit 1-(4-Chlor-phenyl)-piperazin das 1-(4-Chlor-phenyl)-4-[(5-(4-fluorphenyl)-3-pyridyl)-methyl]-piperazin, Dihydrochlorid-Hydrat, F. 135°;
mit 1-(2-Pyridyl)-piperazin das 1-(2-Pyridyl)-4-[(5-(4-fluorphenyl)-3-pyridyl)-methyl]-piperazin, Trihydrochlorid-Dihydrat F. 203°;
mit 1-(2-Pyrimidinyl)-piperazin das 1-(2-Pyrimidinyl)-4-[(5-(4-fluorphenyl)-3-pyridyl)-methyl]-piperazin, Maleat, F. 172°;
mit 1-(3-Trifluormethyl-phenyl)-piperazin das 1-(3-Trifluormethyl-phenylyl)-4-[(5-(4-fluorphenyl)-3-pyridyl)-methyl]-piperazin, Sesquihydrochlorid, F. 237°;
mit 1-(4-Methylcarbonyl-phenyl)-piperazin das 1-(4-Methylcarbonyl-phenyl)-4-[(5-(4-fluorphenyl)-3-pyridyl)-methyl]-piperazin, Sesquihydrochlorid, F. 211°;
mit 1-Phenyl-piperazin das 1-Phenyl-4-[(5-(4-fluorphenyl)-3-pyridyl)-methyl]-piperazin, Dihydrochlorid-Hydrat, F. 207°.

### Beispiel 3

Analog Beispiel 1 erhält man ausgehend von 3-Biphenylylmethylchlorid ("C") [erhältlich z. B. durch radikalische Substitution von 3-Methylbiphenyl] durch Umsetzung mit 1-(2-Methoxy-phenyl)-piperazin das1-(2-Methoxyphenyl)-4-(3-biphenylyl-methyl)-piperazin, Maleat, F. 158-160°.

Analog erhält man durch Umsetzung von "C"
mit 1-Phenyl-piperazin das 1-Phenyl-4-(3-biphenylyl-methyl)-piperazin, Maleat, F. 181-183°;
mit 1-(4-Cyanphenyl)-piperazin das 1-(4-Cyanphenyl)-4-(3-biphenylyl-methyl)-piperazin, F. 139°;
mit 1-(2-Methoxy-5-aminocarbonylphenyl)-piperazin das 1-(2-Methoxy-5-aminocarbonylphenyl)-4-(3-biphenylyl-methyl)-piperazin, Dihydrochlorid, F. 193-196°;
mit 1-(2-Methoxy-5-cyanphenyl)-piperazin das 1-(2-Methoxy-5-cyanphenyl)-4-(3-biphenylyl-methyl)-piperazin, Hydrochlorid, F. 227-229°.

### Beispiel 4

Zu einer Suspension von 200 mg NaOH (fest) in 22 ml Diethylenglykoldimethylether werden bei einer Temperatur von 100° 1,0 g 2-[4-(3-Brombenzyl)-piperazino]-pyrimidin, 0,72 g 4-Trifluormethylbenzolboronsäure, 56 mg Tetrakis-triphenylphosphin-palladium, suspendiert in 10 ml Diethylenglykoldimethylether, unter Rühren hinzugefügt. Danach erwärmt man auf 150° und rührt weitere 1,5 Stunden. Nach Abkühlen versetzt man die Reaktionsmischung mit 50 ml halbkonzentrierter wäßriger HCI-Lösung und extrahiert zweimal mit je 10 ml Dichlormethan, trocknet über Na₂SO₄ und entfernt das Lösemittel. Der Rückstand wird in wenig Ether aufgenommen und über Kieselgel mit Ether/Petrolether im Verhältnis 3:2 chromatographiert. Man erhält 1-(2-Pyrimidinyl)-4-(4'-trifluormethyl-3-biphenylylmethyl)-piperazin als Öl, R_{f} = 0,44 (Ether/Petrolether 3:2). Durch Behandlung mit etherischer HCI-Lösung erhält man das Hydrochlorid von 1-(2-Pyrimidinyl)-4-(4'-trifluormethyl-3-biphenylylmethyl)-piperazin.

### Beispiel 5

Zu einer Lösung von 0,75 ml 3-Bromthiophen in 1 ml Ether werden bei -78° 5,2 ml n-Butyllithium, entsprechend 8,3 mmol, tropfenweise zugegeben und 15 Minuten gerührt. Anschließend fügt man 1,8 g ZnBr₂, suspendiert in 3 ml THF/Ether (1:1) hinzu und läßt auf Raumtemperatur erwärmen. Man rührt 30 Minuten bis zur Zweiphasenbildung, kühlt erneut und fügt 11,2 mg PdCl₂(dppf) hinzu. Man rührt ca. 12 Stunden und läßt dabei die Temperatur auf Raumtemperatur ansteigen. Zur Aufarbeitung säuert man mit 1 molarer HCl-Lösung an, extrahiert zweimal mit je 30 ml Essigsäureethylester, trocknet über Na₂SO₄ und entfernt das Lösemittel. Der Rückstand wird in wenig Ether aufgenommen und über Kieselgel mit Ether/Petrolether im Verhältnis 1:1 chromatographiert. Man erhält 1-(2-Pyrimidinyl)-4-(3-(3-thienyl)-benzyl)-piperazin als öliger Rückstand. Durch Behandlung mit etherischer Maleinsäure-Lösung erhält man das Maleat von 1-(2-Pyrimidinyl)-4-(3-(3-thienyl)-benzyl)-piperazin, F. 208°.

### Beispiel 6

Analog Beispiel 3 erhält man ausgehend von 4'-Trifluormethyl-3-biphenylylmethylchlorid ("G") [erhältlich z.B. durch radikalische Substitution von 4'-Trifluormethyl-4-methylbiphenyl] durch Umsetzung mit 1-(2-Methoxyphenyl)-piperazin das1-(2-Methoxy-phenyl)-4-(4'-Trifluormethyl-3-biphenylyl-methyl)-piperazin.

Analog erhält man durch Umsetzung von "G"
mit 1-Phenyl-piperazin das 1-Phenyl-4-(4'-Trifluormethyl-3-biphenylyl-methyl)-piperazin;
mit 1-(2-Aminocarbonyl-benzofuran-5-yl)-piperazin das 1-(2-Aminocarbonyl-benzofuran-5-yl)-4-(4'-Trifluormethyl-3-biphenylylmethyl)-piperazin;
mit 1-(4-Trifluormethylphenyl)-piperazin das 1-(4-Trifluormethylphenyl)-4-(4'-Trifluormethyl-3-biphenylyl-methyl)-piperazin;
mit 1-(2-Methoxy-5-aminocarbonylphenyl)-piperazin das 1-(2-Methoxy-5-aminocarbonylphenyl)-4-(4'-Trifluormethyl-3-biphenylylmethyl)-piperazin;
mit 1-(2-Methoxy-5-trifluormethylphenyl)-piperazin das 1-(2-Methoxy-5-trifluormethylphenyl)-4-(4'-trifluormethyl-3-biphenylylmethyl)-piperazin.

### Beispiel 7

Eine Lösung von 1,6 g 1-Pyrimidin-2-yl-piperazin in 200 ml THF wird mit 2,75 g 3-Chlor-methyl-4'-trifluormethyl-biphenyl ("H") [erhältlich z. B. durch radikalische Chlorierung von 3-Methyl-4'-trifluor-methylbiphenyl], gelöst in 30 ml THF, versetzt und vier Stunden bei Raumtemperatur gerührt. Nach üblicher Aufarbeitung erhält man das 1-Pyrimidin-2-yl-4-[(4'-trifluormethyl-3-biphenylyl)-methyl]-piperazin.

Analog erhält man durch Umsetzung von "H"
mit 3-Chlormethyl-4'-methoxybiphenyl das 1-Pyrimidin-2-yl-4-[(4'-methoxy-3-biphenylyl)-methyl]-piperazin, Dihydrochlorid, F. 227°;
mit 3-Chlormethyl-2'-fluorbiphenyl das 1-Pyrimidin-2-yl-4-[(2'-fluor-3-biphenylyl)-methyl]-piperazin, Maleat, F. 157°;
mit 3-Chlormethyl-3'-methoxybiphenyl das 1-Pyrimidin-2-yl-4-[(3'-methoxy-3-biphenylyl)-methyl]-piperazin, Maleat, F. 170°;
mit 3-Chlormethyl-2'-methoxybiphenyl das 1-Pyrimidin-2-yl-4-[(2'-methoxy-3-biphenylyl)-methyl]-piperazin, Maleat, F. 145°;
mit 3-Chlormethyl-3'-fluorbiphenyl das 1-Pyrimidin-2-yl-4-[(3'-fluor-3-biphenylyl)-methyl]-piperazin, Maleat, F. 183°;
mit 3-Chlormethyl-4'-fluorbiphenyl das 1-Pyrimidin-2-yl-4-[(4'-fluor-3-biphenylyl)-methyl]-piperazin, Maleat, F. 198°;
mit 3-(2-Thienyl)-benzylchlorid das 1-Pyrimidin-2-yl-4-[3-(2-thienyl)-benzyl]-piperazin, Maleat, F. 181°;
mit 3-(3-Thienyl)-benzylchlorid das 1-Pyrimidin-2-yl-4-[3-(3-thienyl)-benzyl]-piperazin, Maleat, F. 208°;
mit 4-(2-Thienyl)-benzylchlorid das 1-Pyrimidin-2-yl-4-[4-(2-thienyl)-benzyl]-piperazin;
mit 4-(3-Thienyl)-benzylchlorid das 1-Pyrimidin-2-yl-4-[4-(3-thienyl)-benzyl]-piperazin;
mit 2-(2-Thienyl)-benzylchlorid das 1-Pyrimidin-2-yl-4-[2-(2-thienyl)-benzyl]-piperazin;
mit 2-(3-Thienyl)-benzylchlorid das 1-Pyrimidin-2-yl-4-[2-(3-thienyl)-benzyl]-piperazin;
mit 3-(2-Thienyl)- 5-chlormethylpyridin das 1-Pyrimidin-2-yl-4-[3-(2-thienyl)-pyrid-5-yl-methyl]-piperazin;
mit 3-(3-Thienyl)-5-chlormethylpyridin das 1-Pyrimidin-2-yl-4-[3-(3-thienyl)-pyrid-5-yl-methyl]-piperazin.

### Beispiel 8

Eine Gemisch von 0,6 g 1-(2-Methoxy-phenyl)-4-[(5-phenyl-3-pyridyl)-methyl]-piperazin [herstellbar nach Beispiel 1], 1,8 g Pyridinhydrochlorid sowie 50 ml Pyridin wird 3 Stunden gekocht. Man kühlt ab, dampft ein, arbeitet wie üblich auf und erhält 1-(2-Hydroxy-phenyl)-4-[(5-phenyl-3-pyridyl)-methyl]-piperazin.

Analog erhält man durch Etherspaltung
aus 1-(2-Methoxy-phenyl)-4-[(4-phenyl-2-pyridyl)-methyl]-piperazin das 1-(2-Hydroxy-phenyl)-4-[(4-phenyl-2-pyridyl)-methyl]-piperazin;
aus 1-(2-Methoxy-phenyl)-4-[(4-(4-fluorphenyl)-2-pyridyl)-methyl]-piperazin: 1-(2-Hydroxy-phenyl)-4-[(4-(4-fluorpheny))-2-pyridyl)-methyl]-piperazin;
aus 1-(3,5-Dichlor-4-methoxy-phenyl)-4-[(5-(4-fluorphenyl)-3-pyridyl)-methyl]-piperazin 1-(3,5-Dichlor-4-hydroxy-phenyl)-4-[(5-(4-fluorphenyl)-3-pyridyl)-methyl]-piperazin;
aus 1-(4-Methoxy-phenyl)-4-[(5-(4-fluorphenyl)-3-pyridyl)-methyl]-piperazin das 1-(4-Hydroxy-phenyl)-4-[(5-(4-fluorphenyl)-3-pyridyl)-methyl]-piperazin;
aus 1-(3,4-Dimethoxy-phenyl)-4-[(5-(4-fluorphenyl)-3-pyridyl)-methyl]-piperazin das 1-(3,4-Dihydroxy-phenyl)-4-[(5-(4-fluorphenyl)-3-pyridyl)-methyl]-piperazin;
aus 1-(3,5-Dimethyl-4-methoxy-phenyl)-4-[(5-(4-fluorphenyl)-3-pyridyl)-methyl]-piperazin 1-(3,5-Dimethyl-4-hydroxy-phenyl)-4-[(5-(4-fluorphenyl)-3-pyridyl)-methyl]-piperazin;
aus 1-(3-Methoxy-phenyl)-4-[(5-(4-fluorphenyl)-3-pyridyl)-methyl]-piperazin das 1-(3-Hydroxy-phenyl)-4-[(5-(4-fluorphenyl)-3-pyridyl)-methyl]-piperazin;
aus1-(2-Methoxy-phenyl)-4-(3-biphenylyl-methyl)-piperazin das 1-(2-Hydroxy-phenyl)-4-(3-biphenylyl-methyl)-piperazin;
aus 1-(2-Methoxy-5-aminocarbonylphenyl)-4-(3-biphenylyl-methyl)-piperazin das 1-(2-Hydroxy-5-aminocarbonylphenyl)-4-(3-biphenylyl-methyl)-piperazin;
aus 1-(2-Methoxy-5-cyanphenyl)-4-(3-biphenylyl-methyl)-piperazin das 1-(2-Hydroxy-5-cyanphenyl)-4-(3-biphenylyl-methyl)-piperazin;
aus 1-(2-Methoxy-5-aminocarbonylphenyl)-4-(2'-cyan-4-biphenylylmethyl)-piperazin das 1-(2-Hydroxy-5-aminocarbonylphenyl)-4-(2'-cyan-4-biphenylyl-methyl)-piperazin;
aus 1-(2-Methoxy-5-cyanphenyl)-4-(2'-cyan-4-biphenylyl-methyl)-piperazin das 1-(2-Hydroxy-5-cyanphenyl)-4-(2'-cyan-4-biphenylyl-methyl)-piperazin.

### Beispiel 9

Eine Mischung aus 130 mg 1-(3-Biphenylyl)-piperazin, 53 mg 3-Bromanisol, 57 mg Natrium-tert.-butylalkoholat und 8 mg [PdCl₂{P(*o*-Tolyl)₃}₂] in 10 ml Toluol wird 3 Stunden auf 100° erhitzt. Nach Abkühlen auf Raumtemperatur wird die Reaktionsmischung in 40 ml Ether aufgenommen und mit gesättigter NaCl-Lösung gewaschen. Die organische Phase wird abgetrennt, über Na₂SO₄ getrocknet und das Lösemittel entfernt. Der Rückstand wird in wenig Ether aufgenommen und über Kieselgel chromatographiert. Man erhält 1-(3-Methoxyphenyl)-4-(3-biphenylyl)-piperazin als öligen Rückstand, FAB-MS: M⁺+1: 359.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat in 3 I zweifach destilliertem Wasser wird mit 2 n Salzsäure auf pH 6,5 eingestellt, steril filtriert, in Injektionsgläser abgefüllt, lyophilisiert und steril verschlossen. Jedes Injektionsglas enthält 5 mg Wirkstoff.

### Beispiel B: Suppositorien

Man schmilzt ein Gemisch von 20 mg eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 938 g NaH₂PO₄ x 2 H₂O, 28,48 g Na₂HPO₄ x 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose; Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird in Ampullen abgefüllt, unter aseptischen Bedingungen lyophilisiert und steril verschlossen. Jede Ampulle enthält 10 mg Wirkstoff.

## Patentansprüche

1. Piperazin-Derivate der Formel I 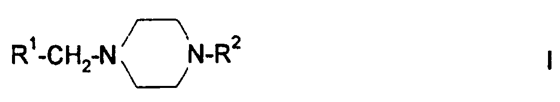 worin
R¹ unsubstituiertes oder einfach durch Ph oder 2- oder 3-Thienyl substituiertes Pyridyl oder Phenyl,
R² Ph' oder Het,
Ph und Ph' jeweils unabhängig voneinander Phenyl, wobei beide Reste jeweils unsubstituiert oder ein-, zwei- oder dreifach durch F, Cl, Br, I, OH, OA, A, CF₃, NO₂, CN, COA, CONH₂, CONHA, CONA₂ oder 2- oder 3-Thienyl substituiert sein können,
Het einen gesättigten, teilweise oder vollständig ungesättigten mono- oder bicyclischen heterocyclischen Rest mit 5 bis 10 Ringgliedern, wobei 1 oder 2 N- und/oder 1 oder 2 O-Atome vorliegen können und der heterocyclische Rest ein- oder zweifach durch F, Cl, Br, I, OA, CF₃, A oder NO₂ substituiert sein kann
und
A Alkyl mit 1 bis 6 C-Atomen
bedeuten
sowie deren physiologisch unbedenkliche Salze.

2. (a) 1-(2-Pyrimidinyl)-4-(3-(3-thienyl]-benzyl)-piperazin;
(b) 1-[5-(4-Fluorphenyl)-pyrid-3-yl-methyl]-4-(2-pyrimidyl)-piperazin;
(c) 4-[4-(3-Biphenylylmethyl)-1-piperazinyl]-benzonitril;
(d) 1-(4-Chlorphenyl)-4-[5-(4-fluorphenyl)-3-pyridylmethyl)-piperazin;
(e) 1-(3'-Fluor-3-biphenylylmethyl)-4-(2-pyrimidinyl)-piperazin;
(f) 2-[4-(5-(3-Thienyl)-3-pyridylmethyl)-1-piperazinyl]-pyrimidin
gemäß Anspruch 1 sowie deren Salze.

3. Verfahren zur Herstellung von Piperazin-Derivaten der Formel I gemäß Anspruch 1 sowie von deren Salzen, **dadurch gekennzeichnet, daß** man eine Verbindung der Formel II 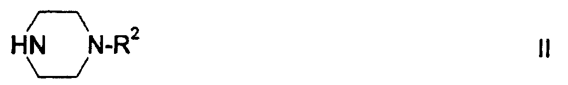 worin R² die angegebene Bedeutung besitzt, mit einer Verbindung der Formel III
R¹-CH₂-L III
worin
L Cl, Br, I, OH, O-CO-A, O-CO-Ph, O-SO₂-Ar, wobei Ar für Phenyl oder Tolyl und A für Alkyl stehen, oder eine andere reaktionsfähig veresterte OH-Gruppe oder leicht nucleophil substituierbare Abgangsgruppe bedeutet und
R¹ die angegebene Bedeutung hat,
umsetzt,
oder daß man eine Verbindung der Formel IV
H₂N-R² IV
worin R² die angegebene Bedeutung hat, mit einer Verbindung der Formel V 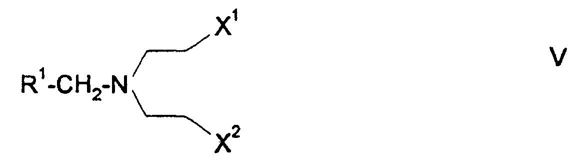 worin X¹ und X² gleich oder verschieden sein können und Cl, Br, I, OH oder eine reaktionsfähige funktionell abgewandelte OH-Gruppe bedeuten, und R¹ die angegebene Bedeutung hat,
umsetzt,
oder daß man eine Verbindung der Formel VI 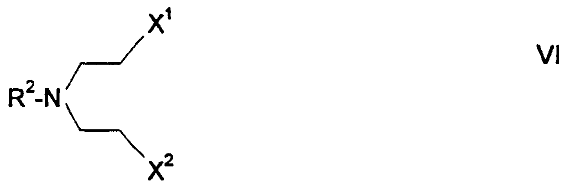 worin R², X¹ und X² die bereits angegebenen Bedeutungen haben,
mit einer Verbindung der Formel VII
R¹-CH₂-NH₂ VII
worin R¹ die angegebene Bedeutung hat
umsetzt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt,
und/oder daß man gegebenenfalls einen Rest R¹ und/oder R² in einen anderen Rest R¹ und/oder R² umwandelt, indem man z.B. eine OA-Gruppe unter Bildung einer OH-Gruppe spaltet und/oder eine CN-, COOH- oder COOA-Gruppe derivatisiert und/oder daß man z.B. ein primäres oder sekundäres N-Atom alkyliert und/oder daß man eine erhaltene Base oder Säure der Formel I durch Behandeln mit einer Säure oder Base in eines ihrer Salze umwandelt.

4. Verfahren zur Herstellung pharmazeutischer Zubereitungen, **dadurch gekennzeichnet, daß** man eine Verbindung der Formel I und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, **gekennzeichnet durch** einen Gehalt an mindestens einer Verbindung der allgemeinen Formel I und/oder einem ihrer physiologisch unbedenklichen Salze.

6. Verwendung von Verbindungen der Formel I nach Patentanspruch 1 oder von deren physiologisch unbedenklichen Salzen zur Herstellung eines Arzneimittels.

7. Verbindungen der Formel I nach Anspruch 1 oder von deren physiologisch unbedenklichen Salzen zur Bekämpfung von Krankheiten.

## Claims

1. Piperazine derivatives of the formula I 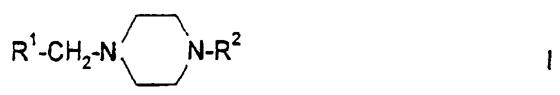 in which
R¹ is pyridyl or phenyl, each of which is unsubstituted or monosubstituted by Ph or 2- or 3-thienyl,
R² is Ph' or Het,
Ph and Ph' are each, independently of one another, phenyl, where both radicals may each be unsubstituted or mono-, di- or trisubstituted by F, Cl, Br, I, OH, OA, A, CF₃, NO₂, CN, COA, CONH₂, CONHA, CONA₂ or 2- or 3-thienyl,
Het is a saturated, partially unsaturated or fully unsaturated mono- or bicyclic heterocyclic radical having from 5 to 10 ring members, in which 1 or 2 N and/or 1 or 2 O atoms may be present, and where the heterocyclic radical may be mono- or disubstituted by F, Cl, Br, I, OA, CF₃, A or NO₂
and
A is alkyl having from 1 to 6 C atoms,
and physiologically acceptable salts thereof.

2. (a) 1-(2-pyrimidinyl)-4-(3-(3-thienyl]benzyl)piperazine;
(b) 1-[5-(4-fluorophenyl)pyrid-3-ylmethyl]-4-(2-pyrimidyl)-piperazine;
(c) 4-[4-(3-biphenylylmethyl)-1-piperazinyl]benzonitrile;
(d) 1-(4-chlorophenyl)-4-[5-(4-fluorophenyl)-3-pyridylmethyl)-piperazine;
(e) 1-(3'-fluoro-3-biphenylylmethyl)-4-(2-pyrimidinyl)piperazine;
(f) 2-[4-(5-(3-thienyl)-3-pyridylmethyl)-1-piperazinyl]pyrimidine;
according to Claim 1, and salts thereof.

3. Process for the preparation of piperazine derivatives of the formula I according to Claim 1 and salts thereof, **characterised in that** a compound of the formula II 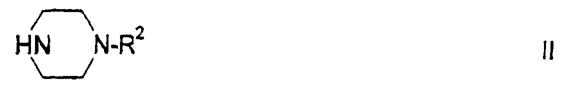 in which R² has the stated meaning, is reacted with a compound of the formula III
R¹-CH₂-L III
in which
L is Cl, Br, I, OH, O-CO-A, O-CO-Ph, O-SO₂-Ar, where Ar is phenyl or tolyl and A is alkyl, or another reactively esterified OH group or leaving group which readily undergoes nucleophilic substitution, and
R¹ has the stated meaning,
or **in that** a compound of the formula IV
H₂N-R² IV
in which R² has the stated meaning, is reacted with a compound of the formula V 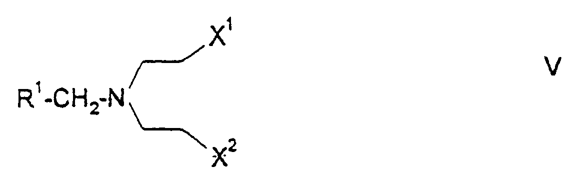 in which X¹ and X² may be identical or different and are Cl, Br, I, OH or a reactive, functionally modified OH group, and R¹ has the stated meaning,
or **in that** a compound of the formula VI 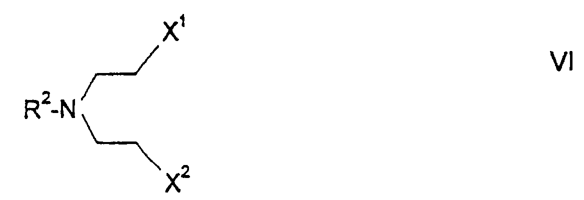 in which R², X¹ and X² have the meanings indicated above,
is reacted with a compound of the formula VII
R¹-CH₂-NH₂ VII
in which R¹ has the stated meaning,
or **in that** a compound which otherwise conforms to the formula I, but which, instead of one or more hydrogen atoms, contains one or more reducible group(s) and/or one or more C-C and/or C-N bond(s), is treated with a reducing agent,
or **in that** a compound which otherwise conforms to the formula I, but which, instead of one or more hydrogen atoms, contains one or more solvolysable group(s), is treated with a solvolysing agent,
and/or **in that**, if desired, a radical R¹ and/or R² is converted into another radical R¹ and/or R² by, for example, cleaving an OA group with formation of an OH group and/or derivatising a CN, COOH or COOA group and/or **in that**, for example, a primary or secondary N atom is alkylated and/or **in that** a base or acid of the formula I obtained is converted into one of its salts by treatment with an acid or base.

4. Process for the preparation of pharmaceutical preparations, **characterised in that** a compound of the formula I and/or one of its physiologically acceptable salts is converted into a suitable dosage form together with at least one solid, liquid or semi-liquid excipient or adjuvant.

5. Pharmaceutical preparation, **characterised by** a content of at least one compound of the general formula I and/or one of its physiologically acceptable salts.

6. Use of compounds of the formula I according to Patent Claim 1 or of physiologically acceptable salts thereof for the preparation of a medicament.

7. Compounds of the formula I according to Claim 1 or physiologically acceptable salts thereof for combating illnesses.

## Revendications

1. Dérivés de pipérazine de formule I 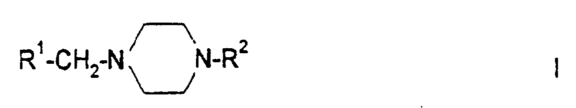 dans laquelle
R¹ représente pyridyle ou phényle, chacun de ceux-ci étant non substitué ou mono-substitué par Ph ou 2- ou 3-thiényle,
R² représente Ph' ou Het,
Ph et Ph' représentent chacun, indépendamment l'un de l'autre, phényle, où les deux radicaux peuvent chacun être non substitués ou mono-, di- ou tri- substitués par F, Cl, Br, I, OH, OA, A, CF₃, NO₂, CN, COA, CONH₂, CONHA, CONA₂ ou 2- ou 3-thiényle,
Het représente un radical hétérocyclique mono- ou bicyclique, saturé, partiellement insaturé ou totalement insaturé, ayant de 5 à 10 chaînons, dans lesquels 1 ou 2 atomes de N et/ou 1 ou 2 atomes d'O peuvent être présents, et où le radical hétérocyclique peut être mono- ou di-substitué par F, Cl, Br, I, OA, CF₃, A ou NO₂
et
A représente un alkyle ayant de 1 à 6 atomes de C,
et des sels physiologiquement acceptables de ceux-ci.

2. (a) 1-(2-pyrimidinyl)-4-(3-(3-thiényl]benzyl)pipérazine;
(b) 1-[5-(4-fluorophényl)pyrid-3-ylméthyl]-4-(2-pyrimidyl)-pipérazine;
(c) 4-[4-(3-biphénylylméthyl)-1-pipérazinyl]benzonitrile;
(d) 1-(4-chlorophényl)-4-[5-(4-fluorophényl)-3-pyridylméthyl)-pipérazine;
(e) 1-(3'-fluoro-3-biphénylylméthyl)-4-(2-pyrimidinyl)pipérazine;
(f) 2-[4-(5-(3-thiényl)-3-pyridylméthyl)-1-pipérazinyl]pyrimidine;
selon la revendication 1, et des sels de ceux-ci.

3. Procédé de préparation de dérivés de pipérazine de formule I selon la revendication 1 et des sels de ceux-ci, **caractérisé en ce qu'**un composé de formule II 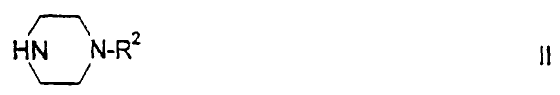 dans laquelle R² a la signification indiquée, est réagi avec un composé de formule III
R¹-CH₂-L III
dans laquelle
L représente Cl, Br, I, OH, O-CO-A, O-CO-Ph, O-SO₂-Ar, où Ar représente phényle ou tolyle et A représente alkyle, ou un autre groupement OH estérifié de manière réactive ou un groupement partant subissant facilement une substitution nucléophile, and
R¹ a la signification indiquée,
ou **en ce qu'**un composé de formule IV
H₂N-R² IV
dans laquelle R² a la signification indiquée, est réagi avec un composé de formule V 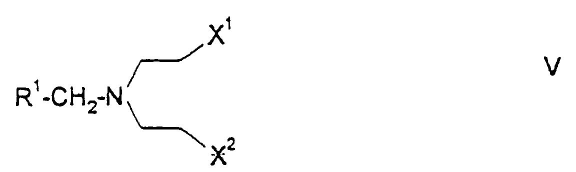 dans laquelle X¹ et X² peuvent être identiques ou différents et représentent Cl, Br, I, OH ou un groupement OH réactif et fonctionnellement modifié, et R¹ a la signification indiquée,
ou **en ce qu'**un composé de formule VI 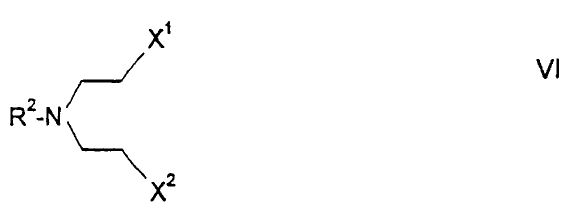 dans laquelle R², X¹ et X² ont les significations indiquées ci-dessus,
est réagi avec un composé de formule VII
R¹-CH₂-NH₂ VII
dans laquelle R¹ a la signification indiquée,
ou **en ce qu'**un composé se conformant par ailleurs à la formule I, mais qui, au lieu d'un ou plusieurs atome(s) d'hydrogène, contient un ou plusieurs groupement(s) pouvant être réduit(s) et/ou une ou plusieurs liaison(s) C-C et/ou C-N, est traité par un agent réducteur,
ou **en ce qu'**un composé se conformant par ailleurs à la formule I, mais qui, au lieu d'un ou plusieurs atome(s) d'hydrogène, contient un ou plusieurs groupement(s) solvolysable(s), est traité par un agent de solvolyse,
et/ou **en ce que**, si on le désire, un radical R¹ et/ou R² est converti en un autre radical R¹ et/ou R², par exemple, par le clivage d'un groupement OA avec la formation d'un groupement OH et/ou la dérivatisation d'un groupement CN, COOH ou COOA et/ou **en ce que**, par exemple, un atome de N primaire ou secondaire est alkylé et/ou **en ce qu'**une base ou un acide de formule I obtenu(e) est converti(e) en l'un de ses sels par traitement par un acide ou une base.

4. Procédé de préparation de préparations pharmaceutiques, **caractérisé en ce qu'**un composé de formule I et/ou l'un de ses sels physiologiquement acceptables est converti en une forme de dosage convenable en association avec au moins un excipient ou adjuvant solide, liquide ou semi-liquide.

5. Préparation pharmaceutique, **caractérisée par** une teneur d'au moins un composé de formule générale I et/ou l'un de ses sels physiologiquement acceptables.

6. Utilisation de composés de formule I selon la revendication 1 ou de sels physiologiquement acceptables de ceux-ci, pour la préparation d'un médicament.

7. Composés de formule I selon la revendication 1 ou sels physiologiquement acceptables de ceux-ci, pour lutter contre des maladies.
